# EUROPÄISCHE PATENTSCHRIFT

(11) **EP 0 460 622 B1**
(45) Veröffentlichungstag und Bekanntmachung des Hinweises auf die Patenterteilung: **31.08.1994**
(21) Anmeldenummer: 91109143.7
(22) Anmeldetag: 04.06.1991
(51) Int. Cl.: A23K 1/18, A61K 31/03, A61K 31/425, A61K 31/435, A61K 31/47, A61K 33/24

(54) **Arzneimittelzubereitung zur systemischen Behandlung von Zier- und Nutzfischen**
Pharmaceutical preparation for the systemic treatment of toy fish and domestic fish
Préparation pharmaceutique pour le traitement systématique des poissons d'agréments et des poissons utiles

(30) Priorität: 05.06.1990 DE 4017964
(43) Veröffentlichungstag der Anmeldung: 11.12.1991
(73) Patentinhaber: Tetra Werke Dr.rer.nat. Ulrich Baensch GmbH, 49324 Melle (DE)
(72) Erfinder: Schmidt, Hartmut, Dr., W-4504 Georgsmarienhütte (DE); Ritter, Günter, Dr., W-4980 Bünde 1 (DE)
(74) Vertreter: Mansmann, Ivo

(56) Entgegenhaltungen:
- WO-A-85/05015
- AT-B- 262 736
- AT-B- 336 385
- DE-A- 1 543 297
- DE-B- 2 009 159
- FR-A- 1 570 434
- GB-A- 1 494 295
- US-A- 2 960 406
- US-A- 3 021 216
- US-A- 3 660 571
- Das Aquarium, 220, 10.87 "Aus der Praxis für die Praxis", L.Zehner
- Treatment of fish parasites, Parasitol Res (1992)78 pages 183-192
- Taschenbuch der Fischkrankheiten, 4. Auflage, 1981, Gustav Fischer Verlag, Stuttgart, Seiten 260-277, 288-311
- Römpps Chemie-Lexikon, 8. Auflage, Band 4, 1985, Franckh'sche Verlagsbuchbandlung, Stuttgart, Seite 2586

## Beschreibung

Die Erfindung betrifft enterale feste Arzneifuttermittelzubereitung für Fische, die mindestens einen der Wirkstoffe Malachitgrün, Brilliantgrün, Gentiana Violett, Kristallviolett, Pararosanilin, Fuchsin, Rivanol, Chinin, Hexamethylentetramin, Übergangsmetallverbindungen und/oder ein quaternäres Ammoniumsalz als Wirkstoff in einer Konzentration von 0,005 bis 10 Gew.% allein oder in Kombination mit anderen Wirkstoffen enthält, wobei die Wirkstoffe derart von den Träger- bzw. Futterstoffen gebunden werden, daß kein Auswaschen durch Wasser stattfindet, sowie Verfahren zu deren Herstellung und deren Verwendung.

Bei der Pflege, Zucht und Hälterung von Süß- und Meerwasserfischen treten in Hobbyaquarien und -teichen und in noch stärkerem Maße in Intensivhälterungsanlagen von Fishfarming und Aquakultur parasitäre Erkrankungen auf, die die befallenen Tiere gefährden und zu erheblichen finanziellen Verlusten führen können. Besonders häufig kommen dabei folgende fischpathogene Organismen vor: Dinoflagellaten: Oodinium pillularis (Süßwasser), Oodinium ocellatum (Meerwasser); Flagellaten: Costia necatrix, Hexamita symphysodonis; Ciliaten: Ichthyophthirius multifiliis, (Süßwasser), Cryptocarion (Meerwasser), Chilodonella, Trichodina (Süßwasser); Monogeneen: Haut- und Kiemensaugwürmer wie z. B. Gyrodactylus oder Dactylogyrus.

Die Behandlung der Ektoparasitosen, d. h. der Erkrankungen mit Parasiten, die auf oder in der Oberhaut der Fische leben, erfolgt bis heute ausschließlich durch die sogenannte Badtherapie, bei der antiparasitäre Wirkstoffe dem Hälterungswasser in geeigneten, bioziden Konzentrationen zugesetzt werden.

Häufig werden dabei nur die freischwimmenden Parasitenstadien, die sogenannten Schwärmer, geschädigt, nicht aber die in der Haut oder unter der Oberhaut festsitzenden Stadien.

Die gesamte Behandlung stützt sich demnach auf die Abtötung der Schwärmer und damit die Verhinderung einer Neu- oder Reinfektion und auf das Immunsystem der Fische, welches die parasitierenden Erregerstadien zusätzlich oder ausschließlich bekämpft.

Nach herrschender Auffassung des Fachleute können ektoparasitäre Erkrankungen nur von außen und nicht durch systemisch verabreichte Heilmittel, z. B. Medizinalfutter behandelt werden.

Dagegen werden endoparasitäre Erkrankungen, z. B. durch Darmparasiten, üblicherweise durch systemisch applizierte Arzneimittelzubereitungen, wie z. B. Medizinalfutter erfolgreich behandelt. (Vgl. N. Herwig, "Handbook of Drugs and Chemicals used in the Treatment of Fish Diseases", S. 128, 136, Charles C. Thomas, Publisher, Sprinfield, Illinois, U.S.A. 1979; E. Amlacher "Taschenbuch der Fischkrankheiten" Gustav Fischer Verlag Stuttgart, 1981; L. Zemer, Das Aquarium, 220, 515, 1987.)

Die Therapie ektoparasitärer Erkrankungen von Zier- und Nutzfischen wird dagegen üblicherweise unter Zugabe von biozid, bzw. antiseptisch wirkenden Farbstoffen, z. B. der Gruppe der Triphenylmethan- und Acridinderivate zum Hälterungswasser durchgeführt. Zu diesen Farbstoffen gehören beispielsweise N-[4-[[4-(Diethylamino)-phenyl]-penylmethylen]-2,5-cyclohexadien-1-yliden]-N-ethyl-ethanaminiumsulfat (Brilliantgrün); N-[4-[[4-(Dimethylamino)-phenyl]-phenylmethylen]-2,5-cyclohexadien-1-yliden]-N-methyl-methanaminiumchlorid oder-oxalat (Malachitgrün); N-[4-[Bis[4-(dimethylamino)-phenyl]-methylen]-2,5-cyclohexadien-1-yliden]-N-methyl-methanaminiumchlorid (Gentiana Violett); 3,6-Diamino-10-methylacridinium-chlorid gemischt mit 3.6-Diamino-acridin (Acriflavin); 9-Amino-acridin und 2-Ethoxy-6,9-diamino-acridinium-lactat-monohydrat (Rivanol) und ggf. deren Leukoformen. Daneben werden (in der Badtherapie) auch antiparasitäre Wirkstoffe aus anderen chemischen Verbindungsklassen eingesetzt, z. B.: Chinolin-derivate wie z.B. Chinin; Quaternäre Ammoniumsalze; Silber- und Kupfersalze; Metallkolloide; Nitrofurane wie z.B. Nifurpirinol; Nitrothiazole wie z.B. 2-Amino-5-nitrothiazol.

Die Badtherapie mit den beschriebener antiparasitären Wirkstoffen ist jedoch mit einer Reihe von Nachteilen behaftet: Parasiten in oder unter der Oberhaut der Fische werden meistens nicht erfasst. Da üblicherweise das gesamte Hälterungswasser (Aquarium, Teich, Intensivanlage) mit dem antiparasitären Wirkstoff versetzt werden muß, ist zudem ein sehr hoher Wirkstoffeinsatz erforderlich. Des weiteren ist die erforderliche Wirkstoffkonzentration schwer einzustellen, da während der Behandlung durch Abbauprozesse (chemisch, biologisch, photochemisch) und Adsorption an Oberflächen nicht berechenbare Wirkstoffverluste eintreten. Häufig stört auch die durch Farbstoffe hervorgerufene intensive Wasserfärbung und die Neigung der Badlösung, verschiedene Materialien anzufärben. Da das gesamte Hälterungssystem betroffen ist, führt die Anwesenheit der bioziden Wirkstoffe zu nicht erwünschten und u. U. sogar schädigenden Nebenwirkungen, wie beispielsweise zur Schädigung von anderen wichtigen Wasserorganismen, z. B. Wasserpflanzen, planktonischen Organismen oder zur Schädigung des biologischen Rasens in Filtersystemen. Damit wird die biologische Abbauleistung geschädigt. Auch wirken die genannten therapeutisch aktiven Verbindungen bei äußerer Anwendung bisweilen kiementoxisch.

Aufgabe der Erfindung war es daher, Möglichkeiten der Therapie von ektoparasitären und ektobakteriellen Erkrankungen bei Fischen zu schaffen, die die genannten Nachteile vermeiden.

Gegenstand der Erfindung ist daher ein Medizinalfutter für Fische sowie dessen Verwendung bei der systemischen Behandlung von ektoparasitären und ektobakteriellen Erkrankungen.

Die geschilderten Nachteile der Badtherapie können durch die erfindungsgemäße, für den Fachmann überraschende Möglichkeit der enteralen, systemischen Behandlung von ektoparasitären und ektobakteriellen Erkrankungen von Zier- und Nutzfischen mit Arzneimittelzubereitungen, insbesondere Medizinalfutter umgangen werden.

Die überraschend gefundene und unerwartet hohe systemische Wirksamkeit bislang nur äußerlich angewandter Wirkstoffe gegen Ektoparasiten und Ektobakterien, d. h. Organismen, die den Fisch auf und/oder unter der äußeren Haut- und Schleimhautschicht befallen haben, bietet bisher nicht realisierbare Therapiemöglichkeiten. Erfindungsgemäß kann nämlich eine gezielte, orale Verabreichung des Medizinalfutters an die Fische erfolgen. Parasiten bzw. Bakterien in oder unter der Oberhaut der Fische werden damit therapeutisch vollständig erfaßt. Die aus dem Medizinalfutter durch das Hälterungswasser erfolgende Wirkstoffextraktion ist sehr gering, und, da zudem die absolut verabreichte Wirkstoffmenge sehr klein ist, wird das Hälterungssystem mit nur vernachlässigbar geringen Wirkstoffmengen belastet. Die toxische Belastung von Wasserorganismen wird dadurch praktisch auf Null reduziert, die Filter-Abbauaktivität wird nicht beeinträchtigt, eine Wasserfärbung ist nicht mehr erkennbar und die kiementoxische Wirkung ist eliminiert. Unkalkulierbare Wirkstoffverluste treten während der Behandlung ebenfalls nicht mehr auf. Ein äußerst erwünschter Nebeneffekt ist jedoch die gleichzeitig erfolgende Bekämpfung von gegebenenfalls vorhandenen Endoparasiten.

Durch die Auswahl geeigneter Wirkstoffe lassen sich mit dem neuartigen Medizinalfutter die nachfolgend als beispielhaft aufgeführten Ektoparasiten bei Zier- und Nutzfischen bekämpfen, nämlich Dinoflagellaten wie Oodinium; Flagellaten wie Costia, (Hexamita); Ciliaten wie Ichthyophthirius, Cryptocarion, Chilodonelle, Trichodina; Monogenea wie Haut- und Kiemensaugwürmer, z. B. Gyrodactylus, Dactylogyrus.

Auch Ektobakteriosen haben sich überraschend als bekämpfbar erwiesen, Z. B. alle Haut- und Flosseninfekte durch gramnegative und grampositive Bakterien, wie Aeromonas, Pseudomonas, Flexibacter.

Als Nebenwirkung werden bei der systemischen Behandlung der Ektoparasiten und Ektobakteriosen natürlich auch alle inneren Parasiten- und Bakterienerkrankungen von Zier- und Nutzfischen systemisch mitbehandelt, falls die eingesetzten Wirkstoffe ein entsprechendes Wirkungsspektrum aufweisen.

Überraschenderweise können zur erfindungsgemäßen systemischen Behandlung von Ektoparasitosen und Ektobakteriosen zunächst alle Wirkstoffe eingesetzt werden, die man bislang entweder ausschließlich zur Badtherapie eingesetzt hat oder in Ausnahmefällen auch schon in Form von enteralen Arzneimittelzubereitungen, jedoch ausschließlich zur Behandlung von inneren Parasitosen, vorzugsweise Darmparasitosen verwendet hat.

Ausschließlich zur Badtherapie wurden bisher eingesetzt: Triphenylmethan-Derivate, wie z.B. Malachitgrün (alle Salze wie z. B. Chlorid, Oxalat, Sulfat), Malachitgrün-Carbinolbase, Brilliantgrün (alle Salze), Kristallviolett (alle Salze), Gentianaviolett (alle Salze), Pararosanilin (alle Salze, Base), Fuchsin (Rosanilin) (alle Salze) sowie alle Leukoformen der Farbstoffkationen. Daneben auch Thionin-Derivate, mit Ausnahme von Methylenblau B. Weiterhin Thionin-Derivate mit kleinerem N-Methylierungsgrad sowie alle Leukoformen der Thionin-Derivate; sowie Nitrothiazol-Derivate, wie z.B. 2-Amino-5-nitrothiazol und Übergangsmetall-Derivate, wie z.B. Cu-, Ag-Salze oder Komplexe, Cu- oder Ag-Metallkolloide; alle mikrobicide Quaternäre Ammoniumsalze.

Darüberhinaus fanden einige Wirkstoffe auch in den bereits erwähnten Arzneimittelzubereitungen Verwendung, die zur peroralen Aufnahme bestimmt, jedoch nicht zur Bekämpfung ektoparasitärer Erkrankungen eingesetzt wurden, da diese Indikation nicht bekannt war. Dies sind Acridin-Derivate, wie z. B. Acriflavin, Methylenblau B, Nitrofuran-Derivate, wie z. B. Nifurpirinol, Enheptin und dessen N-Acetylderivat Acimitrazol sowie Trypaflavin und Acriflavin.

Als Matrix oder Vehikel für die oben beschriebenen, systemisch anzuwendenden anti-ektoparasitären und anti-ektobakteriellen Wirkstoffe können die folgenden Applikationsformen von Trägerbzw. Futtertypen für Zier- und Nutzfische Verwendung finden, nämlich folienartiges Futter, Flockenfutter, pelletiertes Futter, extrudiertes Futter, expandiertes Futter, granuliertes Futter, gemahlenes Futter, pulverisiertes Futter, tablettiertes Futter, Futterpasten und Futtersuspensionen, Futtergelees, Aspikfutter oder sonstige ggf. mit Lockstoffen angereicherte Trägersubstanzen.

Das Einbringen der therapeutischen Wirkstoffe erfolgt in einer technologisch geeigneten Vorstufe im Herstellungsprozess, z.B. im Rohstoff-Pulvergemisch und dessen wasserhaltigen Verfahrensstufen, derart, daß eine möglichst feine und homogene Verteilung der Wirkstoffe im fertiggestellten Futter erreicht wird.

Es hat sich gezeigt, daß die meisten der beschriebenen Wirkstoffe, insbesondere, wenn sie in kationischer Form vorliegen (z.B. Malachitgrün, Acridinium-Salze, quaternäre Ammoniumsalze usw.) sehr stark adsorptiv von den Träger- bzw. Futterstoffen gebunden werden. Die Folge dieser ausgeprägten Wirkstoffimmobilisierung ist, daß kein Auswaschen mehr durch Wasser und kein Abfärben, z.B. auf die Hände des Anwenders erfolgt. Dadurch wird eine hohe Anwendungssicherheit selbst bei humantoxischen oder -gefährdenden Wirkstoffen erreicht und es sind deshalb auch keine Sicherheitsmaßnahmen bei der Produktion, Abfüllung, Lagerung und bei der therapeutischen Anwendung erforderlich.

Ähnliche Vorteile werden auch erreicht, wenn reduzierte Vorstufen der Farbstoffkationen, die sog. Leuko-Formen, oder andere, ebenfalls in Wasser sehr schwer lösliche Derivate eingesetzt werden.

In der nachfolgenden Tabelle I sind beispielhaft für einige Wirkstoffe die Bereiche der Konzentrationen im Futter zusammengestellt. Alle Angaben beziehen sich auf mg Wirkstoff pro kg Futter oder auf Gewichtsprozente.

**TABELLE I**

| Wirkstoff | Konzentrationsbereich | | Bevorzugter Konzentrationsbereich | |
|---|---|---|---|---|
| | mg/kg | % | mg/kg | % |
| Malachitgrün chlorid (u.a. Salze) | 100-10000 | 0,01-1,0 | 200-4000 | 0,02-0,4 |
| Malachitgrün-Carbinolbase | 100-10000 | 0,01-1,0 | 200-4000 | 0,02-0,4 |
| Kristallviolett (Gentianaviolett) | 100-10000 | 0,01-1,0 | 200-4000 | 0,02-0,4 |
| 9-Aminoacridin | 100-50000 | 0,01-5,0 | 500-20000 | 0,05-2,0 |
| Ethacridinlactat (Rivanol) | 200-50000 | 0,02-5,0 | 1000-30000 | 0,1-3,0 |
| Acriflavin (Trypaflavin) | 200-50000 | 0,02-5,0 | 500-30000 | 0,05-3,0 |
| Methylenblau | 200-20000 | 0,02-2,0 | 500-10000 | 0,05-1,0 |

Mit den erfindungsgemäßen Medizinalfuttervarianten können Ektoparasitosen, Ektobakteriosen und als therapeutischer Nebeneffekt auch systemische Erkrankungen durch Endoparasiten und Endobakterien bei allen Fischarten aus der Zierfischgruppe (Süßwasser und Seewasser) und der Nutzfischgruppe (Süßwasser und Seewasser) in allen vorkommenden und gebräuchlichen abgeschlossenen und nicht abgeschlossenen Hälterungssystemen behandelt werden.

Selbst Fische in ihrer natürlichen Umgebung sind bei Futteraufnahme therapierbar.

Die folgenden Wirksamkeitsnachweise und Beispiele sollen die Erfindung näher erläutern, jedoch keinesfalls den Gegenstand der Erfindung einschränken:

### Herstellung von Medizinalfutter:

Ein erfindungsgemäßes Medizinalfutter wird in der dem Fachmann geläufigen Weise hergestellt, indem entweder der gewünschte Wirkstoff der Rohfuttermischung vor der Endverarbeitung zu Flocken, Pellets, Tabletten, Kapseln, Extrudaten etc. in der erforderlichen Menge beigemischt wird, oder das Fertigprodukt des Futters mit einer Lösung des Wirkstoffs besprüht wird und anschließend endgetrocknet wird.
Ein typisches Medizinalfutter enthält 0,005 bis 10% des Wirkstoffs, bevorzugt 0,05 bis 3%.

Für die Wirksamkeitsversuche wurde ein Flockenfutter für Zierfische mit 0,2 % Malachitgrünchlorid angereichert und an ektoparasitär erkrankte Zierfische anstelle des nicht medikierten, zur normalen Fütterung verwendeten Flockenfutters verabreicht. Dabei wurden die folgenden Ergebnisse erzielt.
1. Behandlung von Ichthyophthirius
   30 Prachtschmerlen (Botia macracautha) und 30 Fadenfische (Trichogaster trichopteri), die stark mit Ichthyophthirius befallen waren, wurden einmal täglich mit dem Malachitgrünhaltigen Medizinalfutter gefüttert. Im Vergleich zur unbehandelten Kontrolle wurde innerhalb von fünf Tagen ein 100prozentiger Behandlungserfolg erreicht.
2. Behandlung von Costia und Chilodonella
   40 Rote Neons (Paracheirodon axelrodi), die mit Costia und Chilodo nella doppelt infiziert waren, wurden einmal täglich mit dem Malachitgrün-haltigen Medizinalfutter gefüttert. Im Vergleich zur unbehandelten Kontrolle wurde innerhalb von fünf Tagen ein 100prozentiger Behandlungserfolg erreicht.
3. Andere ektoparasitäre Erkrankungen
   Analog zu den oben erwähnten Beispielen 3.1 und 3.2 konnten mit dem beschriebenen, Malachitgrün-haltigen Medizinalfutter folgende Ektoparasitosen völlig geheilt werden:
   Oodinium pillulare, Trichodina spec. und Gyrodactylus
   Entsprechende Heilungserfolge werden auch mit den anderen, in der Erfindungsbeschreibung aufgeführten Medizinalfuttern erreicht.
   Besonders vorteilhaft bei der Behandlung von Ektoparasitosen mit den beschriebenen Medizinalfuttern ist dabei die völlige Unschädlichkeit: Alle behandelten Fische zeigten keinerlei Anzeichen von Unwohlsein oder Schädigungen während und nach der Behandlung.

Die Wirksamkeit von Triphenylmethanfarbstoffen wie z. B. Brilliantgrün und Malachitgrün gegen grampositive Bakterien macht diese Wirkstoffe in Form der erfindungsgemäßen Medizinalfutter zudem als weitere Anwendungsmöglichkeit zusätzlich geeignet zur Behandlung von externen und internen Bakteriosen mit grampositiven Erregern, z. B. mit Corynebakterium, Nocardia und Mycobacterium.

Die bekannte Wirksamkeit von Kristallviolett und Gentianaviolett gegen innere Wurmerkrankungen beim Menschen und bei Säugetieren läßt sich auch zur Bekämpfung von externen und internen Helminthosen bei Fischen ausnutzen. Behandelbar sind dabei Helminthosen, die hervorgerufen sind durch beispielsweise Monogenea (z.B. Gyrodactylus, Dactylogyrus), Cestoda, Trematoda (z.B. Diplostomum) oder Nematoda (z.B. Capillaria).

Eine weitere, grundsätzliche Möglichkeit einer umfassenden Therapie liegt in der kombinierten Anwendung der erfindungsgemäßen Medizinalfutter und einem Heilmittelbad, bei welchem gleichartige oder andere antiparasitäre oder antibakterielle Wirkstoffe dem Wasser in therapeutisch wirksamer Menge zudosiert wurden. Diese Kombinationstherapie bietet die Möglichkeit, auch die nicht parasitierenden, frei schwimmenden oder festsitzenden (an Pflanzen, Steinen, beliebigen Oberflächen) Erregerstadien zu bekämpfen. Daraus ergeben sich als Vorteile eine partielle oder völlige Desinfektion des Systems, eine Herabsetzung der (Re)-Infektionsrate der Fische durch freischwimmende Erregerstadien sowie eine Bekämpfung der am Fisch parasitierenden Erreger von innen durch das Medizinalfutter und von außen durch das Heilbad.

Zu letzterem muß allerdings beachtet werden, daß natürlich bei der zusätzlichen Wirkstoffapplikation über das Wasser alle eingangs aufgeführten prinzipiellen Nachteile einer Badtherapie wirksam werden.

## Patentansprüche

1. Enterale feste Arzneifuttermittelzubereitung für Fische, dadurch gekennzeichnet, daß diese mindestens einen der Wirkstoffe Malachitgrün, Brilliantgrün, Gentiana Violett, Kristallviolett, Pararosanilin, Fuchsin, Rivanol, Chinin, Hexamethylentetramin, Übergangsmetallverbindungen und/oder ein quaternäres Ammoniumsalz als Wirkstoff in einer Konzentration von 0,005 bis 10 Gew.% allein oder in Kombination mit anderen Wirkstoffen enthält, wobei die Wirkstoffe derart von den Träger- bzw. Futterstoffen gebunden werden, daß kein Auswaschen durch Wasser erfolgt.

2. Zubereitung gemäß Anspruch 1, dadurch gekennzeichnet, daß der Wirkstoffanteil 0,05 bis 3 Gew.% beträgt.

3. Zubereitung gemäß der Ansprüche 1 oder 2, dadurch gekennzeichnet, daß der Wirkstoff Malachitgrün oder ein geeignetes Salz oder Derivat davon ist.

4. Zubereitung gemäß der Ansprüche 1 oder 2, dadurch gekennzeichnet, daß der Wirkstoff Chinin oder ein geeignetes Salz oder Derivat davon ist.

5. Verfahren zur Herstellung einer Arzneimittelzubereitung gemäß Anspruch 1 bis 4, dadurch gekennzeichnet daß entweder (A) der Wirkstoff oder die Wirkstoffkombination der Mischung der Hilfsstoffe vor der Endverarbeitung zu Flocken, Pellets, Tabletten, Kapseln, Extrudaten etc. in der erforderlichen Menge beigemischt wird, oder (B) das Fertigprodukt der Zubereitung mit einer Lösung des Wirkstoffs oder der Wirkstoffkombination besprüht und anschließend endgetrocknet wird.

6. Verwendung von Triphenylmethanderivaten oder Thionin-, Nitrothiazol-, Nitrofuran-, Chinolin-, Acridinderivaten, Aldehyd-abspaltenden Verbindungen, Übergangsmetall-Derivaten, Metallkolloiden, Hexamethylentetramin und/oder quaternären Ammoniumsalzen oder deren Kombinationen zur Herstellung einer enteralen Arzmittelzubereitung, eines Arzneimittelfutters oder von Futtermischungen zur systemischen Vorbeugung oder Bekämpfung ektoparasitärer und ektobakterieller Fischkrankheiten.

7. Verwendung von Chinin in Form seiner Salze allein oder in Kombination mit Wirkstoffen gemäß Anspruch 6 zur Herstellung einer enteralen Arzneimittelzubereitung, eines Arzneimittelfutters oder von Futtermischungen zur systemischen Vorbeugung oder Bekämpfung von Ektoparasitosen oder Ektobakteriosen bei Fischen.

8. Verwendung von Chinin in Form seiner Salze allein oder in Kombination mit Wirkstoffen gemäß Anspruch 6 zur Herstellung einer enteralen Arzneimittelzubereitung, eines Arzneimittelfutters oder von Futtermischungen zur systemischen Vorbeugung oder Bekämpfung von Endoparasitosen oder Endobakteriosen bei Fischen.

## Claims

1. Enteral solid medicinal feed preparation for fish, characterised in that said preparation contains at least one of the active substances malachite green, brilliant green, gentian violet, crystal violet, pararosaniline, fuchsin, rivanol, quinine, hexamethylenetetramine, transition metal compounds and/or a quaternary ammonium salt as an active substance in a concentration of 0.005 to 10 % by weight alone or in combination with other active substances, with the active substances being bound by the carrier or feed substances in such a way that no washing out by water occurs.

2. Preparation according to claim 1, characterised in that the proportion of active substance amounts to 0.05 to 3 % by weight.

3. Preparation according to claims 1 or 2, characterised in that the active substance is malachite green or a suitable salt or derivative thereof.

4. Preparation according to claims 1 or 2, characterised in that the active substance is quinine or a suitable salt or derivative thereof.

5. Process for the preparation of a medicinal preparation according to claim 1 to 4, characterised in that either (A) the active substance or the active substance combination is admixed with the mixture of adjuvant substances before final processing to form flocks, pellets, tablets, capsules, extrudates etc. in the required amount or (B) the finished product of the preparation is sprayed with a solution of the active substance or the active substance combination and is subsequently finally dried.

6. Use of triphenylmethane derivatives or thionine derivatives, nitrothiazole derivatives, nitrofurane derivatives, quinoline derivatives, acridine derivatives, compounds splitting off aldehyde, transition metal derivatives, metal colloids, hexamethylenetetramine and/or quaternary ammonium salts or combinations thereof for the preparation of an enteral medicinal preparation, a medicinal feed or feed mixtures for the systemic prevention or treatment of ectoparasitic and ectobacterial fish diseases.

7. Use of quinine in the form of its salts alone or in combination with active substances according to claim 6 for the preparation of an enteral medicinal preparation, a medicinal feed or feed mixtures for the systemic prevention or treatment of ectoparasitoses or ectobacterioses in fish.

8. Use of quinine in the form of its salts alone or in combination with active substances according to claim 6 for the preparation of an enteral medicinal preparation, a medicinal feed or feed mixtures for the systemic prevention or treatment of endoparasitoses or endobacterioses in fish.

## Revendications

1. Préparation alimentaire médicamenteuse solide à administrer aux poissons par voie entérale, caractérisée en ce que cette préparation contient au moins l'une des substances actives: vert malachite, vert brillant, violet gentiane, violet cristal, pararosaniline, fuschine, rivanol, quinine, hexaméthylènetétramine, des dérivés de métaux de transition et/ou un sel d'ammonium quaternaire comme substance active en une concentration de 0,005 à 10% en poids, seuls ou en combinaison avec d'autres substances actives, les substances actives étant liées aux matières formant support ou aux substances alimentaires de telle façon qu'il ne se produise aucune extraction par lavage à l'eau.

2. Préparation selon la revendication 1, caractérisée en ce que la proportion en substance active atteint 0,05 à 3% en poids.

3. Préparation selon la revendication 1 ou 2, caractérisée en ce que la substance active est le vert malachite ou l'un de ses sels ou l'un de ses dérivés appropriés.

4. Préparation selon la revendication 1 ou 2, caractérisée en ce que la substance active est la quinine ou l'un de ses sels ou dérivés appropriés.

5. Procédé de préparation d'une préparation médicamenteuse selon les revendications 1 à 4, caractérisé en ce que:
(A) la substance active ou la combinaison de substances actives est ajoutée en quantité requise au mélange des substances actives avant le traitement final en flocons, pastilles, comprimés, gélules, extrudat, etc; ou
(B) le produit fini de la préparation est pulvérisé à l'aide d'une solution de la substance active ou de la combinaison des substances actives et il est ensuite soumis à un séchage final.

6. Utilisation de dérivés du triphénylméthane ou de dérivés de thionine, nitrothiazol, nitrofuranne, quinoline, ou acridine, de dérivés libérant des aldéhydes, de dérivés de métaux de transition, de colloïdes métalliques, d'hexaméthylène tétramine et/ou de sels d'ammonium quaternaire et/ou de leurs mélanges pour la fabrication d'une préparation médicamenteuse agissant par voie entérale, d'un aliment médicinal ou de mélanges alimentaires pour la prévention ou la lutte systémique contre les maladies ectoparasitaires et ectobactériennes des poissons.

7. Utilisation de quinine sous la forme de ses sels ou en association avec des substances actives selon la revendication 6, pour la fabrication d'une préparation médicamenteuse par voie entérale, d'un aliment médicamenteux ou de mélanges alimentaires pour la prévention ou la lutte systémique contre les ectoparasitoses ou les ectobactérioses des poissons.

8. Utilisation de quinine sous la forme de ses sels, seule ou en combinaison avec des substances actives selon la revendication 6, pour la fabrication d'une préparation médicamenteuse par voie entérale, d'un aliment médicamenteux ou de mélanges alimentaires pour la prévention ou la lutte systémique contre les endoparasitoses ou les endobactérioses des poissons.
